# EUROPEAN PATENT SPECIFICATION

(11) **EP 1 558 931 B1**
(45) Date of publication and mention of the grant of the patent: **13.06.2007**
(21) Application number: 03779464.1
(22) Date of filing: 04.11.2003
(51) Int. Cl.: G01N 33/53, G01N 33/15, G01N 33/26

(54) **ANALYSIS OF PHARMACEUTICAL SOLUBILITY AND STABILITY**
ANALYSE PHARMAZEUTISCHER LÖSLICHKEIT UND STABILITÄT
ANALYSE DES STABILITE ET SOLUBILITE PHARMACEUTIQUES

(30) Priority: 04.11.2002 US 423366 P; 04.11.2002 US 423365 P
(43) Date of publication of application: 03.08.2005
(73) Proprietor: Transform Pharmaceuticals, Inc., Lexington, MA 02421 (US)
(72) Inventor: CHEN, Hong Ming, Acton, MA 01720 (US); GUZMAN, Hector, Jamaica Plain, MA 02130 (US); GARDNER, Colin, Concord, MA 01742 (US)
(74) Representative: Daniels, Jeffrey Nicholas
(86) International application number: PCT/US2003/035094
(87) International publication number: WO 2004/041202

(56) References cited:
- WO-A-01/51919
- WO-A-03/023394
- US-A- 5 699 934
- US-A- 5 922 616
- US-A- 6 109 717
- US-A1- 2001 044 112
- US-B1- 6 306 659
- US-B1- 6 338 820
- US-B1- 6 485 703
- US-B2- 6 544 193
- D'ANTONA P ET AL: "Rheologic and NMR characterization of monoglyceride-based formulations" J BIOMED MATER RES; JOURNAL OF BIOMEDICAL MATERIALS RESEARCH OCT 2000 JOHN WILEY & SONS INC, NEW YORK, NY, USA, vol. 52, no. 1, October 2000 (2000-10), pages 40-52, XP002389811
- LIPINSKI C A: "DRUG-LIKE PROPERTIES AND THE CAUSES OF POOR SOLUBILITY AND POOR PERMEABILITY" JOURNAL OF PHARMACOLOGICAL AND TOXICOLOGICAL METHODS, ELSEVIER, NEW YORK, NY, US, vol. 44, no. 1, 2000, pages 235-249, XP001097602 ISSN: 1056-8719
- BEVAN C D LLOYD R S: "A high-throughput screening method for the determinations of aqueous drug solubility using laser nephelometry in microtiter plates" ANALYTICAL CHEMISTRY, AMERICAN CHEMICAL SOCIETY. COLUMBUS, US, vol. 72, no. 8, 15 April 2000 (2000-04-15), pages 1781-1787, XP002957441 ISSN: 0003-2700

## Description

### FIELD OF THE INVENTION

This invention is directed to methods, systems, and devices for the screening of formulations for solubility and stability. Specific embodiments of the invention are particularly suited for the preparation and analysis of formulations comprising high viscosity and high concentrations of a compound being studied.

### BACKGROUND OF THE INVENTION

It is well documented that physical and chemical properties, such as stability, solubility, dissolution, permeability, and partitioning of most active pharmaceutical ingredients (APIs) are affected by the pharmaceutical compositions comprising them. For example, the absorption, bioavailability, metabolic profile, toxicity, and potency of a pharmaceutical composition's API can be affected by physical and/or chemical interactions it may have with excipients within the composition. The pharmacological effects of an API may also be affected by the excipients' behavior when contacted with biological environments. For example, the acidic environment of the stomach may have an effect on certain excipients that can promote or diminish the bioavailability of the API with which they are administered.

The excipients that, when combined with an API, form a pharmaceutical composition, can similarly have an effect on the manufacture of the composition, as well as its shelf-life. For example, certain excipients may react or degrade to form products that react with an API when exposed to certain conditions, such as moisture or elevated temperature. For reasons such as these, the development of pharmaceutical compositions, not just APIs themselves, is an important part of pharmaceutical development and research. Furthermore, a goal of formulation development is the discovery of formulations that optimize desired characteristics of a compound-of-interest, such as stability, solubility, and bioavailability under storage and administration conditions.

The discovery of such formulations is a tedious process, and typically involves the assessment of numerous excipients, excipient combinations, and physical conditions. Indeed, although some general rules exist, the effect of excipients and combinations of excipients on the physical and chemical properties of a compound-of-interest is not easily predicted. Moreover, there are over 3,000 excipients to choose from when designing a pharmaceutical composition, each of which interacts differently with other excipients and compounds-of-interest. Because of the many variables involved, industry does not have the time or resources to identify, measure, or exploit interactions between excipients and pharmaceuticals, and thus cannot readily provide optimized pharmaceutical formulations.

Additional factors that slow the development of a pharmaceutical composition include, but are not limited to, difficulties that arise from the handling of some excipients and compounds-of-interest. For example, the accurate handling of fine powders, by weight or volume, and the accurate pipeting or aliquoting of viscous fluids can make the preparation of pharmaceutical compositions a difficult and time-consuming task. These problems become even more daunting when a compound-of-interest is available only in small amounts. By way of illustration, non-aqueous formulations such as gel caps can contain high concentrations of compounds-of-interest, typically over 100 mg/mL in some excipients. However, because many such excipients are highly viscous at room temperature, they are difficult to accurately dispense in small volumes. Moreover, the use of large volumes, which may be necessary to provide reliable concentration data, will require the use of substantially more compound-of-interest than would be required for the development of less concentrated pharmaceutical compositions. It is for reasons such as this that many compounds-of-interest are distributed and administered in standard formulations that are not optimized to provide the most desirable storage and use properties. See e.g., Allen's Compounded Formulations: U.S. Pharmacists Collection 1995 to 1998, ed. Lloyd Allen.

Until now, experiments used to test compositions comprising small amounts of compounds-of-interest have been done manually and in small numbers. This has effectively precluded the preparation and testing of large numbers of excipient combinations that may provide unexpected advantages when used for the manufacture, storage, or administration of a compound-of-interest. A need therefore exists for methods of preparing and testing compositions that contain concentrated amounts of compounds-of-interest. A particular need exists for methods and systems that can be used to prepare compound-of-interest formulations comprising highly viscous excipients.

### SUMMARY OF THE INVENTION

This invention is based, in part, on a discovery of methods that can be used to rapidly obtain information about combinations of various excipients, and that such information may be used to develop formulations of compounds-of-interest that have desirable properties. For example, if a compound-of-interest is an API, desired formulations containing it may have long shelf lives, may deliver the compound-of interest in a bioavailable form, and/or may deliver the compound-of-interest at a controlled rate.

One embodiment of the invention encompasses a method of rapidly and efficiently preparing sample formulations comprised of a compound-of-interest and one or more liquid excipients, determining whether the compound-of-interest is soluble in the excipient(s), and optionally screening or ranking the samples that are soluble based on one or more criteria. Examples of such optional criteria include, but are not limited to, the nature of the samples' reactions to pH, ion concentration, solvent, temperature, and light.

Specific methods of the invention are particularly useful for the preparation and screening of substantially non-aqueous formulations. Others are particularly suited for the preparation and screening of formulations than contain high concentrations of a compound-of-interest. Still others allow the preparation of formulations using highly viscous compounds (e.g., excipients).

The present invention provides methods for the investigation of a small amount (micrograms) or a small volume (microliters) of a substantially non-aqueous formulation, which comprises a high concentration of a compound-of-interest and is highly viscous in nature. The methods enable measurements of the solubility of a formulation at various pH levels, ion concentrations, temperatures, as well as in different solvents and under different light conditions.

### BRIEF DESCRIPTION OF THE FIGURES

Specific embodiments of the invention can be understood with reference to the attached figures.
Figure 1 is a general schematic of an embodiment of the invention
Figure 2 provides a more detailed representation of a specific embodiment of the invention
Figure 3 shows the consistency with which an apparatus of the invention can dispense 0.5 microliters of a liquid with a viscosity of about 400 centipoise
Figure 4 illustrates aspects of a vision station that can be used to identify samples that do not contain solid
Figure 5 shows an array of 10 samples in optically transparent wells, each of which comprises a compound-of-interest and a different liquid excipient. The samples that contain solid (i.e., not completely dissolved) compound-of-interest appear occluded.

### DEFINITIONS

As used herein and unless otherwise indicated, the term "array," when used to refer to a plurality of objects (e.g., samples), means a plurality of objects that are organized physically or indexed in some manner (e.g., with a physical map or within the memory of a computer) that allows the ready tracking and identification of specific members of the plurality. Typical arrays of samples comprise at least 6, 12, 24, 94, 96, 380, 384, 1530, or 1536 samples.

As used herein and unless otherwise indicated, the term "controlled amount" refers to an amount of a compound that is weighed, aliquotted, or otherwise dispensed in a manner that attempts to control the amount of the compound. Preferably, a controlled amount of a compound differs from a predetermined amount by less than about 15, 14, 13, 12, 11, 10, 9, 8, 7, 6, 5, 4, 3, 2, or 1 percent of the predetermined amount. For example, if one were to dispense, handle, or otherwise use 100 micrograms of a compound-of-interest, a controlled amount of that compound-of-interest would preferably weight from about 85 micrograms to about 115 micrograms, from about 90 micrograms to about 110 micrograms, from about 95 micrograms to about 105 micrograms, or from about 99 micrograms to about 101 micrograms. Typically, the precise mass of the controlled amount is determined after being dispensed.

As used herein and unless otherwise indicated, the term "ranking" means to organize, index, or otherwise differentiate members of an array with regard to a specific property. Ranking can be based on qualitative or quantitative determinations, and does not require that each member of the array be assigned a specific rank. For example, the ranking of an array of samples by how much solid compound-of-interest is contained in each may simply constitute the identification of one or more samples within the array that contain or do not contain a solid. In a specific embodiment, however, the ranking of an array of samples constitutes assigning to each sample a number, index, or other representation that is indicative of the nature, amount, or quality of a specific property possessed by that sample with respect to other samples in the array. For example, the ranking of ten samples by how much solid compound-of-interest is contained in each may comprise assigning a number to each of the samples, wherein sample number 1 has the largest amount of solid compound-of-interest, and sample number 10 has the lowest amount of solid compound-of-interest. If two samples among the ten contain the same amount of solid, they can, but need not, be assigned the same rank and potency.

As used herein and unless otherwise indicated, the term "sample" refers to an isolated amount of a compound or composition. A typical sample comprises a controlled amount of a compound-of-interest and a solvent and/or an excipient. Specific samples comprise a compound-of-interest in an amount less than about 25 mg, 1 mg, 500 micrograms, 250 micrograms, 100 micrograms, 50 micrograms, 25 micrograms, 10 micrograms, 5 micrograms, 1 micrograms, or 0.5 micrograms. A sample can be contained in container (e.g., a jar, vial, or well), or can be deposited or adsorbed on a surface. The term "sample" includes replicates, e.g. n = 2, 3, 4, 5, 6 or more.

As used herein and unless otherwise indicated, a composition that is "substantially non-aqueous" comprises less than about 10, 9, 8, 7, 6, 5, 4, 3, 2, 1, or 0.5 weight or volume percent water. Compositions that are substantially non-aqueous include those that are non-aqueous.

As used herein and unless otherwise indicated, a " highly viscous" mixture or excipient has a viscosity at room temperature that is greater than about 100, 200, 300, 400, 500, 600, 700, 800, 900, 1000, 1500, 2000, 2500, 3000, 3500, or 4000 centipoise.

### DETAILED DESCRIPTION OF THE INVENTION

This invention is based, in part, on a discovery that aspects and methods of systems referred to as FAST® and CRYSTALMAX^{™} could be optimized for the high-throughput preparation and analysis of compound-of-interest/excipient mixtures such as, but not limited to, substantially non-aqueous, highly viscous, and highly concentrated mixtures. The methods and systems referred to as FAST® are described in U.S. Patent Application No. 09/628,667, the entirety of which is incorporated herein by reference. The methods and systems referred to as CRYSTALMAX^{™} are described in U.S. Patent Application No. 09/756,092 and International Publication WO01/51919, both of which are incorporated herein in their entireties by reference. Methods and systems of this invention are referred to as SFINX^{™}.

This invention encompasses automated and high-throughput methods of preparing and screening formulations that are substantially non-aqueous and/or comprise one or more liquid or semi-solid (i.e., has a melting point less than 40 degrees C) excipients and a controlled amount of a given compound, referred to herein as a "compound-of-interest." Compounds-of-interest include, but are not limited to, APIs. In several methods of the invention, mixtures of a compound-of-interest and one or more liquid excipients are screened with regard to the solubility of the compound-of-interest in the liquid excipient(s). The mixtures may be further screened with regard to the effect conditions such as, but not limited to, pH, ion concentration, solvent, temperature, and light have on them. In this way, methods of the invention can quickly yield large amounts of information directly relevant to the effective formulation of compounds-of interest. For example, methods of the invention can be used in the development of pharmaceutical compositions that increase the bioavailability, shelf life, or other desired characteristics of an API. The methods of the present invention are specifically applicable to small amounts and small volumes of compounds-of-interest and excipients. A small amount of a compound-of-interest or of an excipient can be about 0.5 micrograms, about 1 microgram, about 5 micrograms, about 10 micrograms, about 15 micrograms, about 20 micrograms, about 25 micrograms, about 50 micrograms, about 100 micrograms, about 250 micrograms, about 500 micrograms, about 1 mg, about 25 mg, or an intermediate amount. A small volume of a compound-of-interest or of an excipient can be about 0.5 microliters, about 1 microliter, about 5 microliters, about 10 microliters, about 25 microliters, about 50 microliters, about 100 microliters, about 250 microliters, about 500 microliters, or an intermediate volume.

Accordingly, a first embodiment of the invention encompasses a method of screening compositions that comprise a compound-of-interest, which comprises: (a) preparing an array of substantially non-aqueous samples, wherein each sample in the array comprises a controlled amount of the compound-of-interest and a liquid excipient, and wherein at least two of the samples differ with respect to the liquid excipient they contain and/or the concentration of the compound-of-interest; (b) identifying samples in the array wherein at least some of the compound-of-interest is dissolved in the liquid excipient; and (c) ranking the identified samples.

A second embodiment of the invention encompasses a method of screening compositions that comprise a compound-of-interest, which comprises: (a) providing a plurality of liquid excipients and/or miscible combinations of liquid excipients; (b) preparing an array of substantially non-aqueous samples by contacting, for each sample, a controlled amount of the compound-of-interest with a liquid excipient or a miscible combination of liquid excipients obtained from the plurality, wherein at least two of the samples differ with respect to the liquid excipient or miscible combination of liquid excipients they contain and/or the concentration of the compound-of-interest; (c) identifying samples in the array wherein at least some of the compound-of-interest is dissolved in the liquid excipient or combination of miscible liquid excipients; and (d) ranking the identified samples.

Various aspects of these and other embodiments of the invention are discussed below.

### COMPOUNDS-OF-INTEREST

Embodiments of this invention can be used for the discovery of useful and novel formulations of a wide variety of compounds-of-interest. Examples of compounds-of interest include, but are not limited to, the active components of pharmaceuticals, dietary supplements, alternative medicines, nutraceuticals, sensory compounds, agrochemicals, consumer formulations, and industrial formulations. Particular compounds-of-interest are active pharmaceutical ingredients (APIs). Specific compounds-of-interest have an aqueous solubility that is less than about 1 mg/mL, less than about 100 micrograms/mL, or less than about 10 micrograms/mL.

Pharmaceuticals are substances that have a therapeutic, disease preventive, diagnostic, or prophylactic effect when administered to an animal or a human, and include prescription and over-the-counter drugs. Some examples of compounds-of interest are listed in 2000 Med Ad News 19:56-60 and The Physicians Desk Reference, 56th ed. (2002). Examples of veterinary pharmaceuticals include, but are not limited to, vaccines, antibiotics, growth enhancing components, and dewormers. Other examples of veterinary pharmaceuticals are listed in The Merck Veterinary Manual, 8th ed., Merck and Co., Inc., Rahway, NJ, 1998; The Encyclopedia of Chemical Technology, 24 Kirk-Othomer (4th ed. at 826); and A.L. Shore and R.J. Magee, Veterinary Drugs in ECT, vol. 21 (2nd ed.) (American Cyanamid Co.).

Dietary supplements are non-caloric or insignificant-caloric substances administered to an animal or a human to provide a nutritional benefit or non-caloric or insignificant-caloric substances administered in a food to impart the food with an aesthetic, textural, stabilizing, or nutritional benefit. Dietary supplements include, but are not limited to, fat binders, such as caducean; fish oils; plant extracts, such as garlic and pepper extracts; vitamins and minerals; food additives, such as preservatives, acidulents, anticaking components, antifoaming components, antioxidants, bulking components, coloring components, curing components, dietary fibers, emulsifiers, enzymes, firming components, humectants, leavening components, lubricants, non-nutritive sweeteners, food-grade solvents, thickeners; fat substitutes; flavor enhancers; and dietary aids, such as appetite suppressants. Examples of dietary supplements are listed in (1994) The Encyclopedia of Chemical Technology, 11 Kirk-Othomer (4th ed. at 805-833). Examples of vitamins are listed in (1998) The Encyclopedia of Chemical Technology, 25 Kirk-Othomer (4th ed. at 1) and Goodman & Gilman's: The Pharmacological Basis of Therapeutics, 9th ed., Joel G. Harman and Lee E. Limbird, eds., McGraw-Hill, 1996 p.1547. Examples of minerals are listed in The Encyclopedia of Chemical Technology, 16 Kirk-Othomer (4th ed. at 746) and "Mineral Nutrients" in ECT 3rd ed., vol 15, pp. 570-603, by C.L. Rollinson and M.G. Enig, University of Maryland.

Alternative medicines are substances, preferably natural substances, such as herbs or herb extracts or concentrates, administered to a subject or a patient for the treatment of disease or for general health or well being, which do not require approval by the FDA. Examples of alternative medicines include, but are not limited to, ginkgo biloba, ginseng root, valerian root, oak bark, kava kava, echinacea, *harpagophyti* radix. Other examples are listed in The Complete German Commission E Monographs: Therapeutic Guide to Herbal Medicine, Mark Blumenthal et al. eds., Integrative Medicine Communications 1998.

Nutraceuticals are foods or food products having both caloric value and pharmaceutical or therapeutic properties. Examples of nutraceuticals include, but are not limited to, garlic, pepper, brans and fibers, and health drinks. Other examples are listed in M.C. Linder, ed. Nutritional Biochemistry and Metabolism with Clinical Applications, Elsevier, New York, 1985; Pszczola et al., 1998 Food technology 52:30-37 and Shukla et al., 1992 Cereal Foods World 37:665-666.

### EXCIPIENTS

Excipients are combined with a compound-of-interest to provide formulations of the invention. Typical excipients are liquids or semi-solids at room temperature, although they may be highly viscous. Semi-solids are defined as materials with a melting point between room temperature and 40 degrees C. As used herein and unless otherwise specified, the term "liquid" includes those materials defined as semi-solids. A liquid excipient, according to the present invention, includes excipients with a melting point below 40 degrees C. Indeed, particular embodiments of this invention provide for the preparation and analysis of formulations using excipients having a viscosity at room temperature that is greater than about 100, 200, 300, 400, 500, 600, 700, 800, 900, 1000, 1500, 2000, 2500, 3000, 3500, or 4000 centipoise. In some cases, such excipients may be necessary to provide formulations that contain desirable concentrations of compounds-of-interest.

Excipients may be novel, but commercially available excipients that are generally recognized as safe (GRAS) are preferably used. Although particular embodiments of the invention do use water as an excipient, typical liquid excipients are substantially non-aqueous or non-aqueous. Examples of excipients that can be used in various embodiments of the invention include, but are not limited to: acidulents, such as lactic acid, hydrochloric acid, and tartaric acid; solubilizing components, such as non-ionic, cationic, and anionic surfactants; absorbents, such as bentonite, cellulose, and kaolin; alkalizing components, such as diethanolamine, potassium citrate, and sodium bicarbonate; anticaking components, such as calcium phosphate tribasic, magnesium trisilicate, and talc; antimicrobial components, such as benzoic acid, sorbic acid, benzyl alcohol, benzethonium chloride, bronopol, alkyl parabens, cetrimide, phenol, phenylmercuric acetate, thimerosol, and phenoxyethanol; antioxidants, such as ascorbic acid, alpha tocopherol, propyl gallate, and sodium metabisulfite; binders, such as acacia, alginic acid, carboxymethyl cellulose, hydroxyethyl cellulose; dextrin, gelatin, guar gum, magnesium aluminum silicate, maltodextrin, povidone, starch, vegetable oil, and zein; buffering components, such as sodium phosphate, malic acid, and potassium citrate; chelating components, such as EDTA, malic acid, and maltol; coating components, such as adjunct sugar, cetyl alcohol, polyvinyl alcohol, carnauba wax, lactose maltitol, titanium dioxide; controlled release vehicles, such as microcrystalline wax, white wax, and yellow wax; desiccants, such as calcium sulfate; detergents, such as sodium lauryl sulfate; diluents, such as calcium phosphate, sorbitol, starch, talc, lactitol, polymethacrylates, sodium chloride, and glyceryl palmitostearate; disintegrants, such as collodial silicon dioxide, croscarmellose sodium, magnesium aluminum silicate, potassium polacrilin, and sodium starch glycolate; dispersing components, such as poloxamer 386, and polyoxyethylene fatty esters (polysorbates); emollients, such as cetearyl alcohol, lanolin, mineral oil, petrolatum, cholesterol, isopropyl myristate, and lecithin; emulsifying components, such as anionic emulsifying wax, monoethanolamine, and medium chain triglycerides; flavoring components, such as ethyl maltol, ethyl vanillin, fumaric acid, malic acid, maltol, and menthol; humectants, such as glycerin, propylene glycol, sorbitol, and triacetin; lubricants, such as calcium stearate, canola oil, glyceryl palmitosterate, magnesium oxide, poloxymer, sodium benzoate, stearic acid, and zinc stearate; solvents, such as alcohols, benzyl phenylformate, vegetable oils, diethyl phthalate, ethyl oleate, glycerol, glycofurol, for indigo carmine, polyethylene glycol, for sunset yellow, for tartazine, triacetin; stabilizing components, such as cyclodextrins, albumin, xanthan gum; and tonicity components, such as glycerol, dextrose, potassium chloride, and sodium chloride; and mixtures thereof. Other excipients, such as binders and fillers, are known to those of ordinary skill in the art. See, e.g., Remington's Pharmaceutical Sciences, 18th ed., ed. Alfonso Gennaro, Mack Publishing Co. Easton, PA, 1995; Handbook of Pharmaceutical Excipients, 3rd Edition, ed. Arthur H. Kibbe, American Pharmaceutical Association, Washington D.C. 2000.

Formulations can comprise one or more excipients. For example, a formulation can comprise one, two, three, four, five, or more excipients, in addition to a compound-of-interest. A binary formulation comprises two excipients and a compound-of interest, a ternary formulation comprises three excipients and a compound-of-interest, and so on.

Additional non-limiting examples of excipients include: acetylated monoglycerides, C8/C10 diesters of propylene glycol of coconut oil, caprylic/capric triglyceride, castor oil, coconut oil, com oil, cottonseed oil, diacetylated monoglycerides, ethylene glycol, gelucire 33/01, glycerin, glyceryl linoleate, glyceryl oleate, glyceryl ricinoleate, hydrogenated coconut oil, linoleic acid, mineral oil, mono-/diglyceride from coconut oil (C8/C 10); monoolein:propylene glycol (90:10), myristyl alcohol, oleic acid, olive oil, palm oil, peanut oil, PEG 60 almond glycerides, PEG-6 isostearate, PEG-8 caprylic/capric glyceride, caprylocaproyl macrogol-8 glycerides, poloxamer 331, PEG 1000, PEG 200, PEG 300, PEG 400, PEG 600, polyglycerol-3-diisostearate, polyglycerol-6 dioleate, polyoxyethylene glycerol trioleate, polyoxyl 30 castor oil, polyoxyl 35 castor oil, polyoxyl 40 castor oil, polyoxyl 40 stearate, polypropylene glycol 2000, polypropylene glycol 725, polysorbate 20, polysorbate 40, polysorbate 60, polysorbate 80, propylene glycol, propylene glycol monocaprylate, propylene glycol monolaurate, safflower oil, sesame oil, sorbitan monolaurate, sorbitan monooleate, sorbitan trioleate, soybean oil, sunflower seed oil, triacetin, triethanolamine, trilaurin, water, benzyl alcohol, benzyl benzoate, diethylene glycol monoethyl ether, ethylene glycol monoethyl ether, isopropanolamine, cetearyl alcohol, cetyl alcohol, cetyl esters wax, acetic acid, ethanol, cyclodextrin, poloxamer 188, and sodium hydroxide.

The present invention includes formulations comprising a drug and any one, any two, any three, any four, any five of the excipients listed herein (e.g., above). Each combination is included as an individual specie of the present invention. Each species may also be excluded from the present invention.

### PREPARATION OF AN ARRAY

In a typical embodiment of the invention, each sample in an array comprises at least two components: a compound-of-interest and a liquid excipient. Samples may also comprise one or more solid excipients. In a typical array, samples will differ from each other by the concentration of the compound-of-interest and/or the excipient(s) they contain. In certain circumstances, each sample within an array will differ from the others. In other circumstances, it may be desirable to provide redundancy within the array to reduce experimental error.

The samples of specific arrays comprise less than about 25 mg, 1 mg, 500 micrograms, 250 micrograms, 100 micrograms, 50 micrograms, 25 micrograms, 10 micrograms, 5 micrograms, 1 microgram, or 0.5 micrograms of the compound-of-interest. The samples of specific arrays may comprise less than about 500 microliters, 250 microliters, 100 microliters, 50 microliters, 25 microliters, 10 microliters, 5 microliters, 1 microliter, or 0.5 microliters of liquid excipient(s). Particular arrays comprise samples that contain high concentrations of compound-of-interest, e.g. concentrations greater than about 1 mg/mL, about 10 mg/mL, about 25 mg/mL, about 50 mg/mL, about 100 mg/mL, about 200 mg/mL, about 300 mg/mL about 400 mg/mL, about 500 mg/mL, or an intermediate concentration. In typical embodiments of the invention, containers, such as tubes, vials, or wells, are used to hold the samples. In addition, one or more of the containers are typically provided as controls, to aid in the automated screening of arrays. Typical controls will contain no sample, will contain only compound-of-interest, or will contain only excipient(s).

Each of the samples is prepared by adding a controlled amount of one or more solids (e.g. compound-of-interest and optional solid excipients) to each container (except, perhaps, one or more used as controls), after which a controlled amount of one or more liquids (e.g., one or more liquid excipients) is added to each container. Of course, the particular order in which solids and liquids are combined can be varied as desired. For example, one or more liquid excipients can be added before the controlled amount of one or more solids is added, or the controlled amount of one or more solids can be dispensed before contacting with one or more liquid excipients. Stir bars or tumblers can optionally be added to each container to ensure that its contents are well mixed. Samples may also be heated to help the compound-of-interest in each sample dissolve-to the extent possible-in the liquid excipient(s). In several embodiments of the invention, the formation of arrays is done using automated techniques that allow the rapid preparation of large arrays of samples.

If the compound-of-interest is a liquid at room temperature, it can be transferred to the tubes, wells, or other containers forming an array using conventional techniques (e.g., using a pipettor). If it is a viscous liquid, a preferred method to transfer it to a well or tube comprises the use of a positive displacement pump. Other pump types can be used, however, positive displacement pumps were found to have an unexpectedly high reproducibility in dispensing small volumes of viscous liquid. The controlled transfer of a viscous liquid may be facilitated by a means for heating the pump and transfer lines while in the pump and/or while being transferred. In one embodiment, the pump and transfer lines are heated using heating coils placed around the pump and tubes, along the path of the liquid. For example, greater than 20 percent, 30 percent, 40 percent, 50 percent, 60 percent, 70 percent, 80 percent, 90 percent, or greater than 90 percent of the pump and transfer lines are contacted with a heating means. Some or all of the transfer lines can be contacted with a heating means. In another embodiment, after dispensing the desired amount of liquid, a brief duration of low pressure is applied to each transfer line sufficient to pull a majority or substantially all of the liquid not dispensed into the well (i.e., hanging from the outside of the transfer tip), back into the transfer tip. This low pressure causes any liquid remaining on the tip of the dispenser to return to the interior of the transfer tip, so as to prevent over-dispensing of the desired liquid. This technique is especially useful for manipulating liquids with low surface tension (i.e., less than about 40 dynes/cm). Solid compounds-of-interest can be transferred using conventional methods, although preferred techniques are disclosed in U.S. Provisional Application Nos. 60/423,377, 60/424,001, and 60/430,089, filed on November 4, 2002, November 6, 2002, and December 2, 2002, respectively, the entireties of which are incorporated herein by reference.

Solid and liquid excipients can be transferred to the tubes, wells, or other containers forming an array using the same methods and devices used to transfer the compound-of-interest. In another embodiment of the invention, a plurality of containers are filled with liquid excipients before the array is prepared. Containers may also be charged with mixtures of miscible excipients. Such mixtures are preferably prepared (and their miscibility confirmed) prior to array preparation. In this way, combinations that are not miscible can be determined and excluded from use without incurring the unnecessary waste of the compound-of-interest. In another embodiment, the containers found to contain miscible and otherwise suitable solvents are physically moved (e.g., robotically) to provide an array, or palate, of solvents that can be used for sample array preparation.

In another embodiment, each of the excipient containers is attached to a pump or similar device that can be used to dispense controlled amounts of excipient into the wells of a sample array. In one embodiment, the dispensing of excipients is done using automated means, allowing for the rapid and automated preparation of samples.

### SOLUBILITY DETERMINATION

After samples containing the compound-of-interest and one or more liquid excipients are prepared, they are analyzed to determine the solubility of the compound-of-interest. However, before the solubility analysis is performed, the samples are preferably sealed and then stored, or incubated, under conditions sufficient to ensure that the compound-of-interest in each sample has had an adequate opportunity to dissolve into the liquid excipient(s) to the fullest extent possible. Examples of sufficient conditions include, but are not limited to, agitation (e.g., stirring or sonication) and heating for an adequate amount of time.

Samples within an array can be screened for solubility in a variety of ways. Some methods are automated, and one method, for example, utilizes imaging equipment and software. In one embodiment of this method, the containers (e.g., tubes, vials, or wells) that hold each sample in an array are transparent (i.e., they do not entirely block the transmission of visible or infrared light). After incubation, they are loaded into an imaging station, wherein light is shown through each sample and detected using a digital camera. The image obtained for each sample is compared with that of a control container, which does not contain a solid, and those that appear free of solid are identified.

Other techniques may also be used to detect whether or not a solid compound-of interest is dissolved in a liquid excipient. For example, turbidity measurements are well suited for such measurements, and can be readily automated. Other well known methods can be used to provide more detailed information about how much of a compound-of interest is dissolved in an excipient. Examples include, but are not limited to, high performance liquid chromatography (HPLC), UV spectroscopy, fluorescence spectroscopy, gas chromatography, optical density, and colorimetry. In another embodiment of the invention, samples are filtered or centrifuged to ensure that their liquid portions are free of solids before they are analyzed. Additional methods of determining solubility, and the nature of solubility itself, are well known. See, e.g., Streng et al., 1984 J. Pharm. Sci. 63:605; Kaplan, Drug Metab. Rev. 1:15 (1972); Remington's Pharmaceutical Sciences, pp. 1456-1457 (18th ed., 1995); or Fiese et al., The Theory and Practice of Industrial Pharmacy, pp. 185-188 (3rd ed., 1986).

### SCREENING AND RANKING

According to this invention, samples within an array are initially screened and ranked during the determination of which, if any, of them contain no solid (e.g., solid compound-of-interest). Further characterization of the samples can be used to rank them more completely. In particular, samples that contain only liquid can be analyzed using methods such as, but not limited to, HPLC, Raman spectroscopy (e.g., resonance Raman spectroscopy), and absorption/transmission spectroscopy to determine if the compound-of-interest has undergone any chemical degradation. At the same time, samples that contain some solid can be processed to separate their solid and liquid portions (e.g., by filtering or centrifuge), and the characteristics of one or both portions can be used to further rank the samples. Methods such as, but not limited to, HPLC, nuclear magnetic resonance (NMR) (e.g., ¹H and ¹³C NMR), Raman spectroscopy, and absorption and emission spectroscopy (e.g., infrared, visible, and ultraviolet absorption and emission) can be used to analyze the liquid portion of each sample, for example to determine how much of the compound-of-interest it contains, and whether it has undergone any chemical degradation. Similarly, methods such as, but not limited to, HPLC, NMR, Raman spectroscopy, X-ray spectroscopy, powder X-ray diffraction, absorption and emission spectroscopy, birefringence screening, differential scanning calorimetry (DSC), thermogravimetric analysis (TGA), and particle size measurements can be used to determine the physical and chemical nature of the samples' solid portions.

Samples can also be ranked by the rate at which their solid portions (e.g., solid compound-of-interest) dissolve in their liquid portions (e.g., liquid excipients). For this information, new arrays of samples are prepared under conditions that allow for the kinetic measurement of solubility. In one example, the rate at which a solid compound-of-interest will dissolve in the liquid excipient of a particular sample is determined by preparing a new array of samples, each comprising a controlled amount of compound-of interest. A controlled amount of the liquid excipient is added to each of the samples in the new array, but at different times. For example, at time 0, liquid is added to sample number 1; at time 1, liquid is added to sample number 2; and so on. At a final time, the solid-if any-and liquid portions of each sample in the new array are separated (e.g., by filtration or centrifuge), and the amount of solid for each is determined and correlated with time. By creating such a new array for each sample in the original array, kinetic information is obtained for each of their liquid excipients.

Certain embodiments of the invention rank dissolved samples by observing their behavior when subjected to one or more conditions to which an optimal formulation of the compound-of-interest would be subjected. Examples of such conditions include, but are not limited to, changes in pH, ion concentration, temperature, exposure to particular solvents, and exposure to particles of the compound-of-interest or other compounds that may, for example, catalyze the crystallization of the compound-of-interest. For example, samples can be contacted with an aqueous solution comprising surfactants and having a pH of approximately that of the human stomach (e.g., about 1.5), and the effect of that contact evaluated. Other biological fluids (e.g., blood and physiological saline) can similarly be emulated using solutions and techniques known in the art to determine their effect on a particular formulation of a compound-of-interest. Examples of effects that can be used to rank samples include, but are not limited to, physical effects (e.g., precipitation, phase separation, formation of emulsions or micelles) or chemical effects (e.g., degradation) of the compound-of-interest. Samples that yield a precipitate can be further ranked with regard to the amount of the compound-of-interest precipitated, the speed with which the precipitation occurs, the size of the particles precipitated, and the nature of such particles (e.g., amorphous or crystalline, and if crystalline, their crystal form, or habit). Such information is particularly relevant to the development of safe and effective oral dosage forms of APIs, but is also relevant to the formulation of other compounds-of-interest.

In various embodiments of the invention, pH buffers can be used to simulate a variety of different environments to which a formulation may be exposed. Such environments include, but are not limited to, processing conditions, storage conditions, and in vivo conditions. Examples of pH buffers have pHs of from about 9.0 to about 1.0, from about 7.5 to about 5.0, and from about 6.8 to about 5.5. Other examples have pHs of from about 6.5 to about 10.0, from about 7.5 to about 9.0, and from about 9.0 to about 8.5. Similarly, exposure to UV, visible, and IR light can be used to simulate processing and storage conditions, as can elevated temperatures (e.g., temperatures ranging from about 25 degrees C to about 60 degrees C, from about 30 degrees C to about 50 degrees C, and from about 35 degrees C to about 45 degrees C).

Samples can also be screened and ranked with regard to their chemical or physical stability. For example, samples can be ranked by how much, if any, of the compound-of-interest decomposes as a function of time, either with or without exposure to conditions such as those described above. Samples can also be ranked according to their physical stability, for example, their resistance to phase separation or their tendency to form a precipitate. In this way, information can be rapidly and efficiently obtained that is useful in developing a formulation that has a long shelf-life.

Another embodiment of the invention emcompasses using the methods of the present invention to determine the existence of a synergistic effect of mixtures of excipients on the solubility of a compound-of-interest. Further included are compositions made by the same methods. This embodiment provides a method to determine which mixtures of excipients yield unexpectedly high or low solubility. For example, if the solubility of a compound-of-interest is 5 mg/mL in excipient A, the solubility of the compound-of-interest is 1 mg/mL in excipient B, and the solubility of the compound-of-interest in a mixture of excipients A and B is 15 mg/mL, the discovery of the mixture comprising A and B can be useful in the design of a formulation for the compound-of interest. Similarly, a synergistic effect which decreases the solubility can also be of particular importance. The ability to change the solubility of a compound-of-interest simply by changing the excipient mixture used is a powerful tool in the quest for improved formulations. Synergistic effects of other characteristics such as hygroscopicity, dissolution, and stability can also be found using methods of the present invention. Further included in the present invention are methods and arrays wherein an excipient which does not have an affect on solubility in one sample has an affect on solubility when added with a second excipient. In one embodiment the affect is an increase and in another embodiment the affect is a decrease in solubility. In another embodiment, methods and arrays are included wherein an excipient decreases solubility when combined with a first excipient and increases solubility when combined with a second excipient. Further included are methods and arrays wherein at least two, three, four or more than four different samples comprising at least two, three, four, or more than four excipients have an increase in solubility and a further embodiment is wherein at least two, three, four or more than four samples comprise one, two, three, four or more than four excipients wherein the combination synergisticly increases solubility.

### EXEMPLIFICATION

### Example 1

### Preparation and Screening Arrays

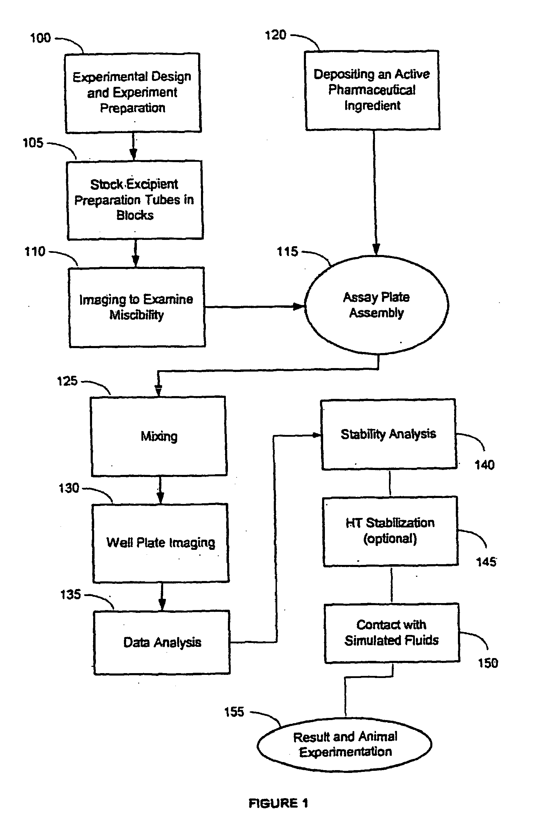

The implementation of one embodiment of the invention can be understood with regard to Figure 1. As represented by step 100 of the figure, an experiment is designed using data known about the compound-of-interest and excipients to be used. In particular, an attempt is made to provide excipients in which the compound-of-interest may be soluble, thereby avoiding waste. In step 105, excipients are placed in containers from which they can be readily dispensed during the sample array preparation. If combinations of excipients are used, they are imaged as indicated in step 110 to confirm that they are miscible before they are actually used. The sample array is prepared by depositing a controlled amount of compound-of-interest into each well of a multi-well plate (step 120), and dispensing a controlled amount of one or more liquid excipients into each well (step 115). The wells are then sealed and mixed (step 125), after which they are imaged to determine which contain dissolved compound-of-interest (step 130). An analysis of this data can be used to prepare new arrays if more information is desired (step 135). In this particular embodiment, the stability of the compound-of-interest/excipient combinations is determined (step 140). Optionally, a stabilizer or an additive (e.g., antioxidant) can be added to minimize chemical degradation (step 145). Next, they are contacted with a simulated biological fluid (step 150). Once enough data has been obtained to prepare a formulation having the desired properties (e.g., compound-of-interest concentration, stability, and bioavailability), formulations are prepared on a larger scale and tested using animal models (step 155).

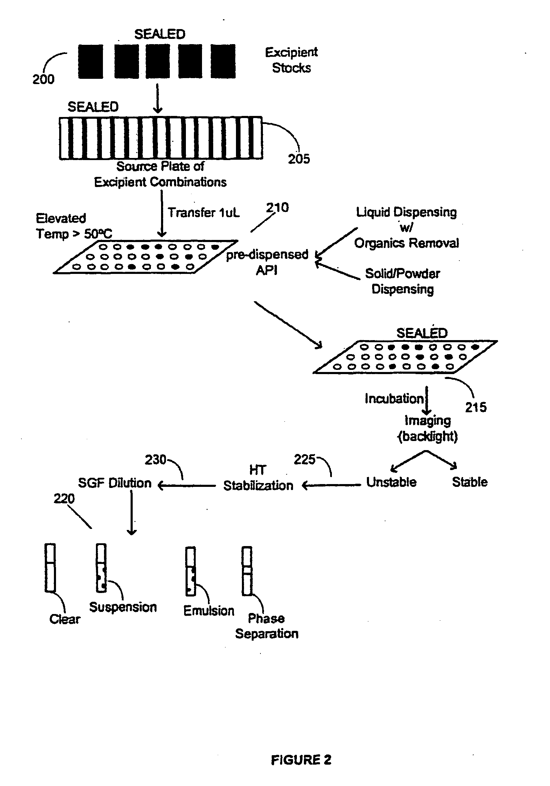

Figure 2 provides a schematic of a specific example of the invention. First, a source plate, or palate, of excipients (205) is prepared from a stock of excipients (200). The source plate comprises liquid excipients contained, for example, in glass tubes or jars held in a block made of a material that is preferably thermally conductive (e.g., a metal such as, but not limited to, aluminum). The source plate may further comprise miscible combinations of two or more liquid excipients and one or more solid excipients dissolved in a liquid excipient. In such cases, the miscibility and stability (e.g., reluctance to separate or form a precipitate) of such combinations is determined before the source plate is used.

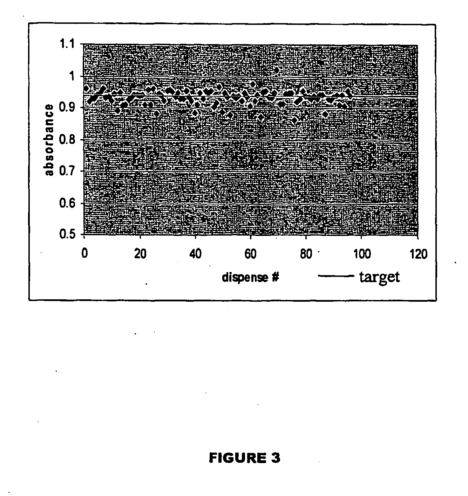

In this example, a syringe-pump-based dispenser, e.g., a synQUAD^{™} (Cartesian Technologies, Inc., Irvine, CA) automated liquid handler, optionally enclosed in a heated box, is used to prepare the source plate (205). Examples of other dispensers that can be used include, but are not limited to, piston-pump-based dispensers (e.g., from IVEK Corporation, North Springfield, VT) and other pump-based dispensers (e.g., from Oyster Bay Pump Works, Hicksville, NY). As shown in Figure 3, the use of such dispensers, heated transfer lines, and a heated source plate can be used to accurately and repeatedly dispense small amounts of viscous excipients.

Referring to Figure 2, once the source plate has been prepared, an array of samples is prepared in, for example, clear-bottom Greiner 384-well plates. The samples are prepared using a programmable multi-channel pipettor, such as the Tecan GenMate^{™} (Tecan US, Research Triangle, NC) or Hydra^{™} (Robbins Scientific, Sunnyvale, CA) robotic liquid transfer station, which may be heated to aid in the transfer of viscous excipients, to dispense a controlled amount (e.g., 5 microliters) of excipient from the source plate (step 210). Each of the wells, apart from one or more used as controls, already contains a controlled amount (e.g., 50 micrograms) of solid compound-of-interest and chemically-resistant small magnetic mixing dowels or mixing disks (e.g., from V&P Scientific, Inc., San Diego, CA). The wells are sealed, after which the samples are then mixed and incubated at room temperature or at an elevated temperature for a suitable amount of time (*e.g*., 30-60 minutes) (step 215).

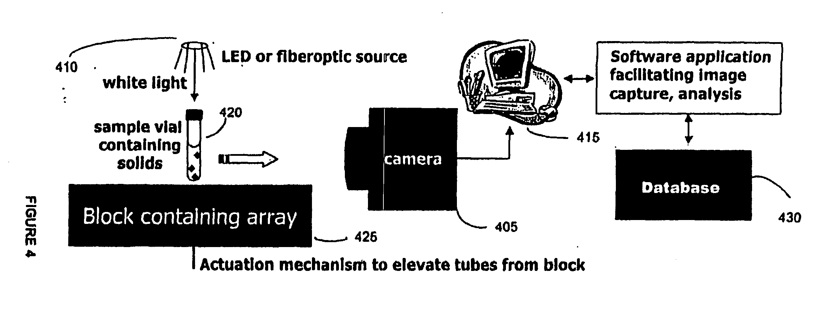

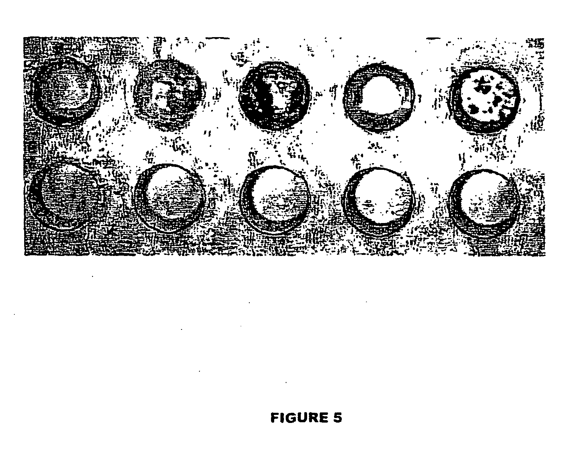

Whether the samples are contained in vials, wells, or some other type of container, each is analyzed after incubation. Samples may be analyzed at this point in order to determine whether any degradation of the compound-of-interest occurred during incubation (e.g., using NMR, optical absorption or emission spectroscopy, or Raman spectroscopy). Optionally, if degradants are present, a stabilizer can be added (step 225). At the very least, samples are analyzed to determine whether or not they contain solid. This is preferably done using an automated imaging, or vision, station, such as that represented in Figure 4. In this embodiment, a light source (410) shines light (e.g., visible or infrared) through each sample container. If the containers are vials held in an opaque block (425), a robotic mechanism is used to remove each vial from the block, position it in front of the light source (410) and digital camera (405), or something that receives light and transmits it to the camera (e.g., a fiberoptic receiver). In a specific embodiment, a Panasonic color camera coupled to an Olympus microscope is used to detect light transmitted through the samples. If the containers are wells, the light source is typically positioned under or above each well, and the camera or receiving device is positioned onthe opposite side of the well. In this way, images such as those shown in Figure 5 can be obtained. Using image-recognition software, such images can be evaluated by computer (415), and the sample containers that are apparently free of solid matter identified for further screening, if so desired. Referring to Figure 4, the identification is typically in the form of a computer database (430), which will contain information associated with a sample at a specific location within the block, plate, or other container used to hold the array of sample containers.

The sample (420) is typically positioned between the light source (410) and the camera (405). However, if the camera is used to detect light scattered from particulate matter within the sample, instead of obtaining an image, it is preferably positioned at a right angle to the light source.

Once the samples that do not contain solid are identified, they are screened and ranked depending on criteria relevant to the intended use of the compound-of-interest formulation being developed. In this example, the data obtained are used in the development of an oral dosage form that provides an API in a bioavailable form. Consequently, one microliter of each solid-free sample is transferred into a tube containing about 500 microliters of a liquid that simulates gastric fluids. The resulting array of tubes is analyzed for physical changes such as those shown in Figure 2 (220), and the samples that correspond to each of the tubes are ranked accordingly. An analysis is also completed over time to determine formulation characteristics such as dissolution and changes in solution state (e.g., precipitation, phase separation, etc.). In this example, samples that remain clear and free of precipitate are ranked higher than those that provide a solution with separate phases or that contain a precipitate, suspension, or emulsion. Samples that formed a suspension of fine particles of the compound-of interest are ranked higher than those that contain a precipitate. Samples that formed a precipitate are ranked with regard to the size of the particles of the precipitate, with the ones providing the largest particles ranked lowest. Particle sizes are estimated using a Zetasizer^{™} 3000 (Malvern Instruments, Inc., Southborough, MA), although other methods, such as laser light scattering, Raman spectroscopy, or X-ray powder diffraction may also be used. The amorphous or crystalline nature of the particles, which can be determined using automated Raman spectroscopy techniques, can also be used to rank the samples. Finally, samples that contain or provide a solution that contains degraded compound-of-interest are ranked below those that do not contain degradants.

### Example 2

### Simulation of In Vivo Conditions

In other embodiments of this invention, a sample (e.g., a mixture of a compound-of-interest and at least one liquid excipient) is exposed to an environment that simulates biological conditions. In this way, the effect of those conditions on a particular formulation can be determined and used to design formulations that are tailored to behave in particular ways when administered to patients by specific routes. Compositions that emulate biological conditions are well known in the art. Examples include, but are not limited to, those disclosed herein.

In one embodiment, a sample is contacted with a liquid that simulates gastric and/or small intestine conditions in the fed and/or fasted states. In the fasted state, the liquid simulating gastric conditions comprises:

| | |
|---|---|
| HCl | 0.01 - 0.05 N |
| Triton x 100 | 1.0 G |
| NaCl | 2.0 G |
| Distilled Water (q.s.) | 1000 mL |

In the fed state, the lumenal composition in the stomach is highly dependent on the composition of the meal ingested. A suitable starting point would be to homogenize the meal to be used in the clinical studies used to study a given formulation of a compound-of-interest with the coadministred volume of water, and measure the pH, buffer capacity, and osmolarity. The use of long-life milk and Intralipid^{®} may also be used.

In another embodiment, a sample is contacted with a liquid that simulates intestinal media. Key differences between gastric and intestinal conditions are the presence of bile and pancreatic enzymes and the higher pH. A medium simulating the fasted state conditions of the small intestine comprises:

| | |
|---|---|
| KH₂PO₄ | 0.029 M |
| NaOH (q.s.) | pH of about 6.3 - 6.8 (preferably 6.5) |
| Sodium taurocholate | 5 mM |
| Lecithin | 1.5 mM |
| KCl | 0.22 M |
| Distilled Water (q.s.) | 1000 mL |

Particular media that simulate the fasted state conditions of the small intestine have a pH of about 6.8, an osmolarity of about 280 to about 310 mOsm, and a buffer capacity of about 10±2 mEq/L/pH.

A medium simulating the fed state conditions of the duodenum comprises:

| | |
|---|---|
| Acetic acid | 0.144 M |
| NaOH (q.s.) | pH 5 |
| Sodium taurocholate | 15mM |
| Lecithin | 4 mM |
| KCl | 0.19 M |
| Distilled Water (q.s.) | 1000 mL |

Particular media that simulate the fed state conditions of the duodenum have a pH of about 5.0, an osmolarity of about 485 to about 535 mOsm, and a buffer capacity of about 72±2 mEq/L/pH. If the formulation contains fatty excipients (e.g., soft gelatin used in capsule formulations) and/or the compound-of-interest is very lipophilic, it may be worth considering the addition of mono- and diclycerides and lipase to the medium to simulate oily phase partitioning and digestion.

The scope of the appended claims should not be limited to the description of the embodiments of the invention described herein.

## Claims

1. A method of screening compositions that comprise a compound-of-interest, which comprises:
(a) preparing an array of substantially non-aqueous samples, wherein each sample in the array comprises a controlled amount of the compound-of-interest and a liquid excipient, and wherein at least two of the samples differ with respect to the liquid excipient they contain and/or the concentration of the compound-of-interest, and further wherein each sample has a concentration greater than 1 mg/ml and a viscosity greater than 0,1 Pas (100 centipoise);
(b) using a positive displacement pump to dispense less than 250 microliters of an excipient with a viscosity of at least 0,1 Pas (100 centipoise);
(c) identifying samples in the array wherein at least some of the compound-of-interest dissolved in the liquid excipient; and
(d) ranking the identified samples.

2. The method of claim 1, further comprising:
(a) providing a plurality of liquid excipients and/or miscible combinations of liquid excipients;
(b) preparing an array of substantially non-aqueous samples by contacting, for each sample, a controlled amount of the compound-of-interest with a liquid excipient or a miscible combination of liquid excipients obtained from the plurality, wherein at least two of the samples differ with respect to the liquid excipient or miscible combination of liquid excipients they contain and/or the concentration of the compound-of-interest, and wherein each sample has a concentration greater than 1 mg/ml and a viscosity greater than 0,1 Pas (100 centipoise); and
(c) identifying samples in the array wherein at least some of the compound-of-interest dissolved in the liquid excipient or combination of miscible liquid excipients.

3. The method of claim 2 wherein the samples in the array that comprise decomposed or degraded compound-of-interest are excluded from the identified samples.

4. The method of claim 2 wherein each of the samples is non-aqueous.

5. The method of claim 2 wherein the samples are identified using HPLC, NMR, IR spectroscopy, UV-visible spectroscopy, Raman spectroscopy, visual imaging, or turbidity measurements.

6. The method of claim 2 wherein the identified samples are ranked by the amount of compound-of-interest dissolved.

7. The method of claim 2 wherein the identified samples are ranked by the degree of decomposition or degradation of the compound-of-interest.

8. The method of claim 2 wherein the identified samples are ranked by contacting each of the identified samples with a solution having a pH of from about 9.0 to about 1.0 to provide a set of modified identified samples, and determining how much compound-of-interest is dissolved in each of the modified identified samples.

9. The method of claim 2 wherein the identified samples are ranked by contacting each of the identified samples with a solution having a pH of from about 9.0 to about 1.0 to provide a set of modified identified samples, and determining a characteristic of any undissolved compound-of-interest in each of the modified identified samples.

10. The method of claim 8 or claim 9 wherein the pH is from about 7.5 to about 5.0.

11. The method of claim 10 wherein the pH from about 6.8 to about 5.5.

12. The method of claim 9 wherein the characteristic is average particle size, polymorph, crystal habit, or chemical purity.

13. The method of claim 12 wherein the characteristic is determined using Raman spectroscopy. X-ray spectroscopy, powder X-ray diffraction, differential scanning calorimetry, thermogravimetric analysis, light-scattering, microscopy, birefringence measurements, NMR, or HPLC.

14. The method of claim 2 wherein the array comprises at least 94 samples.

15. The method of claim 14 wherein the array comprises at least 380 samples.

16. The method of claim 2 wherein the compound-of-interest is an active pharmaceutical agent.

## Patentansprüche

1. Verfahren zum Screenen von Zusammensetzungen, die eine interessierende Verbindung umfassen, welches Verfahren Folgendes umfasst:
(a) das Vorbereiten einer Reihe von im Wesentlichen nichtwässrigen Proben, wobei jede Probe in der Reihe eine kontrollierte Menge der interessierenden Verbindung und einen flüssigen Arzneimittelträger umfasst und wobei sich zumindest zwei der Proben hinsichtlich des flüssigen Arzneimittelträgers, den sie enthalten, und/oder der Konzentration der interessierenden Verbindung unterscheiden und wobei weiters jede Probe eine Konzentration von über 1 mg/ml und eine Viskosität von über 0,1 Pas (100 Centipoise) aufweist;
(b) das Verwenden einer Verdrängerpumpe, um weniger als 250 Mikroliter eines Arzneimittelträgers mit einer Viskosität von mindestens 0,1 Pas (100 Centipoise) abzugeben;
(c) das Identifizieren von Proben in der Reihe, bei denen zumindest etwas von der interessierenden Verbindung im flüssigen Arzneimittelträger gelöst ist; und
(d) das Einstufen der identifizierten Proben.

2. Verfahren nach Anspruch 1, weiters umfassend:
(a) das Bereitstellen einer Mehrzahl von flüssigen Arzneimittelträgern und/oder mischbaren Kombinationen von flüssigen Arzneimittelträgern;
(b) das Vorbereiten einer Reihe von im Wesentlichen nichtwässrigen Proben, dadurch, dass für jede Probe eine kontrollierte Menge der interessierenden Verbindung mit einem flüssigen Arzneimittelträger oder einer mischbaren Kombination von flüssigen Arzneimittelträgern, die aus der Mehrzahl erhalten wurden, in Kontakt gebracht wird, wobei sich zumindest zwei der Proben hinsichtlich des flüssigen Arzneimittelträgers oder der mischbaren Kombination von flüssigen Arzneimittelträgern, den bzw. die sie enthalten, und/oder der Konzentration der interessierenden Verbindung unterscheiden und wobei jede Probe eine Konzentration von über 1 mg/ml und eine Viskosität von über 0,1 Pas (100 Centipoise) aufweist; und
(c) das Identifizieren von Proben in der Reihe, bei denen zumindest etwas von der interessierenden Verbindung im flüssigen Arzneimittelträger oder in der Kombination von mischbaren flüssigen Arzneimittelträgern gelöst ist.

3. Verfahren nach Anspruch 2, wobei die Proben in der Reihe, die eine zersetzte oder abgebaute interessierende Verbindung umfassen, von den identifizierten Proben ausgeschlossen werden.

4. Verfahren nach Anspruch 2, wobei jede Probe nichtwässrig ist.

5. Verfahren nach Anspruch 2, wobei die Proben unter Verwendung von HPLC, NMR, IR-Spektroskopie, UV-Spektroskopie, Raman-Spektroskopie, optischer Bildgebung oder Trübungsmessungen identifiziert werden.

6. Verfahren nach Anspruch 2, wobei die identifizierten Proben hinsichtlich der Menge an gelöster interessierender Verbindung eingestuft werden.

7. Verfahren nach Anspruch 2, wobei die identifizierten Proben hinsichtlich des Zersetzungs- oder Abbaugrads der interessierenden Verbindung eingestuft werden.

8. Verfahren nach Anspruch 2, wobei die identifizierten Proben eingestuft werden, indem jede identifizierte Probe mit einer Lösung, die einen pH-Wert von etwa 9,0 bis etwa 1,0 aufweist, in Kontakt gebracht wird, um einen Satz modifizierter identifizierter Proben bereitzustellen, und indem festgestellt wird, wie viel von der interessierenden Verbindung in jeder modifizierten identifizierten Probe gelöst ist.

9. Verfahren nach Anspruch 2, wobei die identifizierten Proben eingestuft werden, indem jede identifizierte Proben mit einer Lösung, die einen pH-Wert von etwa 9,0 bis etwa 1,0 aufweist, in Kontakt gebracht wird, um einen Satz modifizierter identifizierter Proben bereitzustellen, und indem eine charakteristische Eigenschaft jeglicher ungelösten interessierenden Verbindung in jeder modifizierten identifizierten Probe bestimmt wird.

10. Verfahren nach Anspruch 8 oder Anspruch 9, wobei der pH-Wert etwa 7,5 bis etwa 5,0 beträgt.

11. Verfahren nach Anspruch 10, wobei der pH-Wert etwa 6,8 bis etwa 5,5 beträgt.

12. Verfahren nach Anspruch 9, wobei es sich bei der charakteristischen Eigenschaft um die durchschnittliche Partikelgröße, den Polymorphismus, den Kristallhabitus oder die chemische Reinheit handelt.

13. Verfahren nach Anspruch 12, wobei die charakteristische Eigenschaft unter Verwendung von Raman-Spektroskopie, Röntgenspektroskopie, Pulverröntgendiffraktometrie, Differentialscanningkalorimetrie, thermogravimetrischer Analyse, Lichtstreuung, Mikroskopie, Doppelbrechungsmessungen, NMR oder HPLC bestimmt wird.

14. Verfahren nach Anspruch 2, wobei die Reihe mindestens 94 Proben umfasst.

15. Verfahren nach Anspruch 14, wobei die Reihe mindestens 380 Proben umfasst.

16. Verfahren nach Anspruch 2, wobei die interessierende Verbindung ein pharmazeutischer Wirkstoff ist.

## Revendications

1. Procédé de criblage de compositions comprenant un composé d'intérêt, qui comprend :
(a) la préparation d'une matrice d'échantillons essentiellement non aqueux, dans laquelle chaque échantillon de la matrice comprend une quantité maîtrisée du composé d'intérêt et un excipient liquide, et dans laquelle au moins deux des échantillons diffèrent en ce qui concerne l'excipient liquide qu'ils contiennent et/ou la concentration du composé d'intérêt et dans laquelle, en outre, chaque échantillon a une concentration supérieure à 1 mg/ml et une viscosité supérieure à 0,1 Pa.s (100 centipoises) ;
(b) l'utilisation d'une pompe volumétrique pour distribuer moins de 250 microlitres d'un excipient présentant une viscosité d'au moins 0,1 Pa.s (100 centipoises) ;
(c) l'identification des échantillons de la matrice dans lesquels au moins une partie du composé d'intérêt est dissoute dans l'excipient liquide ; et
(d) le classement des échantillons identifiés.

2. Procédé selon la revendication 1, comprenant en outre :
(a) la fourniture d'une pluralité d'excipients liquides et/ou de combinaisons miscibles d'excipients liquides ;
(b) la préparation d'une matrice d'échantillons essentiellement non aqueux par la mise en contact, pour chaque échantillon de la matrice, d'une quantité maîtrisée du composé d'intérêt et d'un excipient liquide ou d'une combinaison miscible d'excipients liquides choisie dans la pluralité de combinaisons, dans laquelle au moins deux des échantillons diffèrent en ce qui concerne l'excipient liquide ou la combinaison miscible d'excipients liquides qu'ils contiennent et/ou la concentration du composé d'intérêt, et dans laquelle chaque échantillon a une concentration supérieure à 1 mg/ml et une viscosité supérieure à 0,1 Pa.s (100 centipoises) ; et
(c) l'identification des échantillons de la matrice dans lesquels au moins une partie du composé d'intérêt est dissoute dans l'excipient liquide ou dans la combinaison d'excipients liquides miscibles.

3. Procédé selon la revendication 2, dans lequel les échantillons de la matrice qui comprennent le composé d'intérêt décomposé ou dégradé sont exclus des échantillons identifiés.

4. Procédé selon la revendication 2, dans lequel chacun des échantillons est non aqueux.

5. Procédé selon la revendication 2, dans lequel les échantillons sont identifiés par CLHP, RMN, spectroscopie infrarouge, spectroscopie UV-visible, spectroscopie Raman, imagerie visuelle ou par des mesures de la turbidité.

6. Procédé selon la revendication 2, dans lequel les échantillons identifiés sont classés en fonction de la quantité de composé d'intérêt dissoute.

7. Procédé selon la revendication 2, dans lequel les échantillons identifiés sont classés en fonction du degré de décomposition ou de dégradation du composé d'intérêt.

8. Procédé selon la revendication 2, dans lequel les échantillons identifiés sont classés en mettant en contact chacun des échantillons identifiés avec une solution ayant un pH allant d'environ 9,0 à environ 1,0 pour fournir une série d'échantillons identifiés modifiés, et en déterminant la quantité de composé d'intérêt dissoute dans chacun des échantillons identifiés modifiés.

9. Procédé selon la revendication 2, dans lequel les échantillons identifiés sont classés en mettant en contact chacun des échantillons identifiés avec une solution ayant un pH allant d'environ 9,0 à environ 1,0 pour fournir une série d'échantillons identifiés modifiés, et en déterminant une caractéristique d'un composé d'intérêt non dissous dans chacun des échantillons identifiés modifiés.

10. Procédé selon la revendication 8 ou la revendication 9, dans lequel le pH va d'environ 7,5 à environ 5,0.

11. Procédé selon la revendication 10, dans lequel le pH va d'environ 6,8 à environ 5,5.

12. Procédé selon la revendication 9, dans lequel la caractéristique est la taille moyenne de particules, le polymorphisme, le comportement cristallin ou la pureté chimique.

13. Procédé selon la revendication 12, dans lequel la caractéristique est déterminée par spectroscopie Raman, spectroscopie aux rayons X, diffraction des rayons X sur poudre, calorimétrie différentielle à balayage, analyse thermogravimétrique, diffusion de la lumière, microscopie, mesures de la biréfringence, RMN ou CLHP.

14. Procédé selon la revendication 2, dans lequel la matrice comprend au moins 94 échantillons.

15. Procédé selon la revendication 14, dans lequel la matrice comprend au moins 380 échantillons.

16. Procédé selon la revendication 2, dans lequel le composé d'intérêt est un agent pharmaceutique actif.
